# EUROPÄISCHE PATENTSCHRIFT

(11) **EP 4 148 038 B1**
(45) Veröffentlichungstag und Bekanntmachung des Hinweises auf die Patenterteilung: **10.01.2024**
(21) Anmeldenummer: 21196659.3
(22) Anmeldetag: 14.09.2021
(51) Int. Cl.: C07C 67/303, C07C 69/75

(54) **VERFAHREN ZUR KERNHYDRIERUNG VON DIALKYLTEREPHTHALATEN MIT GERINGER NEBENPRODUKTBILDUNG**
METHOD FOR THE RING HYDROGENATION OF DIALKYL TEREPHTHALATES WITH LOW BY-PRODUCT FORMATION
PROCÉDÉ D'HYDROGÉNATION DU NOYAU DES DIALKYLTÉRÉPHTALATES À FAIBLE FORMATION DE SOUS-PRODUITS

(43) Veröffentlichungstag der Anmeldung: 15.03.2023
(73) Patentinhaber: Evonik Oxeno GmbH & Co. KG, 45772 Marl (DE)
(72) Erfinder: ZANTHOFF, Horst-Werner, 45481 Mülheim a. d. Ruhr (DE); BAUER, Julia, 45721 Haltern am See (DE); GRASS, Michael, 45721 Haltern am See (DE); SCHNEIDER, Thomas, 46514 Schembeck (DE)
(74) Vertreter: Evonik Patent Association

(56) Entgegenhaltungen:
- US-A1- 2006 167 151
- US-A1- 2021 032 189

## Beschreibung

Die vorliegende Erfindung betrifft ein Verfahren zur Kernhydrierung von Dialkylterephthalaten mit C3- bis C16-Alkylgruppen in einer Hydriereinheit aus zwei hintereinandergeschalteten Reaktionseinheiten. Bei dem erfindungsgemäßen Verfahren wird ein geeigneter Verfahrensparameter in Bezug zur ersten Reaktionseinheit so geregelt, dass ein bestimmter Reaktionsumsatz erreicht wird.

Weichmacher werden in vielen technischen Bereich eingesetzt, um Kunststoffe wie Polyvinylchlorid (PVC) weicher und elastischer zu machen. Seit vielen Jahren sind Phthalate, also die Diester der (ortho-) Phthalsäure, die dominierende Weichmacherklasse. In den letzten Jahren haben aber auch die Alkylester von Cyclohexandicarbonsäuren an Bedeutung gewonnen, nicht zuletzt wegen der Diskussion um eventuelle Gesundheitsbedenken gegen die Phthalat-basierten Weichmacher. Dabei spielen vor allem die 1,2-Cyclohexandicarbonsäuredialkylester und neuerdings auch die 1,4-Cyclohexandicarbonsäuredialkylester eine Rolle.

1,2- und 1,4-Cyclohexandialkyldicarbonsäureester können durch die Hydrierung (wird nachfolgend synonym mit dem Begriff Kernhydrierung verwendet) des aromatischen Rings der entsprechenden Phthalate oder Terephthalate hergestellt werden. Entsprechende Kernhydrierungen werden für die Phthalate, also die Dialkylphthalate, heutzutage bereits großtechnisch eingesetzt, beispielsweise bei der Umsetzung von DINP (Diisononylphthalat) zu DINCH (1,2-Diisononylcyclohexandicarbonsäureester). Hinsichtlich der Hydrierung der Terephthalate, also der Dialkylterephthalate, geht man davon aus, dass solche Verfahren wirtschaftlich uninteressanter sind, weil die Reaktion der Dialkylterephthalate langsamer verläuft und sich dabei mehr Nebenprodukte bilden als bei der Kernhydrierung der Dialkylphthalate. Die Dokumente US2021032189 und US2006167151 offenbaren ein Verfahren zur Kernhydrierung von 1,2-Dialkylphthalate und 1,4-Dialkylterephthalate unter Verwendung mehrerer in Reihe geschalteter Reaktoren.

Es bestand daher die Aufgabe, ein verbessertes Verfahren zur Kernhydrierung von Dialkylterephthalaten bereitzustellen, welches auch im Vergleich zur Kernhydrierung von Dialkylphthalaten wirtschaftlich betrieben werden kann. Die Aufgabe der vorliegenden Erfindung bestand deshalb darin, ein Verfahren zur Kernhydrierung von Dialkylterephthalaten in einer Hydriereinheit bereitzustellen, welches eine effizientere Produktion der entsprechenden Ester ermöglicht.

Die Aufgabe konnte durch das in Anspruch 1 beschriebene Verfahren zur Kernhydrierung von Dialkylterephthalaten mit C3- bis C16-Alkylgruppen gelöst werden. Bevorzugte Ausgestaltungen dieses Verfahrens sind in den Unteransprüchen angegeben.

Das erfindungsgemäße Verfahren ist demnach ein Verfahren zur Kernhydrierung von Dialkylterephthalaten mit C3- bis C16-Alkylgruppen, vorzugsweise Dialkylterephthalaten mit C4- bis C11-Alkylgruppen, zu den 1,4-Cyclohexandicarbonsäureestern mit den entsprechenden Alkylgruppen in einer Hydriereinheit aus zwei hintereinandergeschalteten Reaktionseinheiten, die aus einem Reaktor oder mehreren parallelen Reaktoren bestehen, die in Kreislauffahrweise oder in geradem Durchgang betrieben werden, wovon der oder die Reaktor(en) in der zweiten Reaktionseinheit im geraden Durchgang betrieben werden, wobei in den Reaktoren der zwei Reaktionseinheiten der Hydriereinheit jeweils ein heterogener Hydrierkatalysator vorliegt und wobei das Verfahren sich dadurch auszeichnet, dass
der eingesetzte aromatische Carbonsäureester als Feed in den Reaktoren der Reaktionseinheiten mit einem Wasserstoff-enthaltenden Gas in Kontakt gebracht wird, wobei der Reaktionsumsatz in der ersten Reaktionseinheit überwacht wird, und
wobei mindestens ein Parameter in Bezug zur ersten Reaktionseinheit, ausgewählt aus der Gruppe, bestehend aus der der ersten Reaktionseinheit zugeführten Feedmenge (Gesamtmenge aus Frischfeed und Recycle), der Menge an zu hydrierendem Dialkylterephthalat, der Reaktortemperatur in dem oder den Reaktoren der ersten Reaktionseinheit, dem Temperaturanstieg, dem Kreislaufvolumenstrom, dem Feed/Recycle Verhältnis, dem Verhältnis von Wasserstoff zu aromatischem Carbonsäureester, dem Reaktordruck, der Feedzusammensetzung (bspw. Inhibierung der Reaktion durch Zugabe bspw. von Substanzen, die die Aktivität des Katalysators gezielt reduzieren), der Katalysatormenge, der Katalysatorzusammensetzung, sofern eine Kühlung vorhanden ist, die Menge an umgewälztem Kühlmittel oder der Menge an Produkt im Austrag der ersten Reaktionseinheit und eine Kombination davon so geregelt wird, dass ein Reaktionsumsatz im Bereich von 85 bis 93 %, vorzugsweise 88 bis 92 % erzielt wird.

Es wurde überraschenderweise gefunden, dass eine Begrenzung des Reaktionsumsatzes in der ersten Reaktionseinheit dazu führt, dass die Menge an Nebenprodukten und/oder die Menge an Edukt im finalen Hydrierprodukt (nach Durchlauf durch die zweite Reaktionseinheit) reduziert werden kann. Das erhaltene Reaktionsprodukt ist also reiner und muss weniger aufwändig aufgereinigt werden oder liegt sofort in der notwendigen Spezifikation vor, so dass es ohne weiteren Aufreinigungsaufwand verkauft oder weiterverwendet werden kann.

Die Hydriereinheit besteht erfindungsgemäß aus zwei hintereinandergeschalteten Reaktionseinheiten, die jeweils aus einem Reaktor oder mehreren parallelen Reaktoren bestehen. In der zweiten Reaktionseinheit werden der oder die Reaktor(en) im geraden Durchgang betrieben. Der oder die Reaktor(en) in der ersten Reaktionseinheit können auf verschiedene Art betrieben werden. Es ist jedoch bevorzugt, wenn in der ersten Reaktionseinheit ein Reaktor vorhanden ist, dieser im Kreislauf betrieben wird. Die Einstellung des Reaktionsumsatzes erfolgt dann in Bezug auf Eingangsfeed und Eingang in die zweite Reaktoreinheit.

Der Parameter, der für die Begrenzung des Umsatzes in dem oder in den parallelen Reaktoren der ersten Reaktionseinheit geregelt wird, wird im Rahmen der vorliegenden Erfindung vorzugsweise aus der Gruppe, bestehend aus der dem mindestens einen Kreislaufreaktor zugeführten Feedmenge (Gesamtmenge aus Frischfeed und Recycle), der Menge an zu hydrierenden Dialkylterephthalat, der Reaktortemperatur in dem mindestens einen Kreislaufreaktor, dem Temperaturanstieg (= Differenz zwischen der Temperatur am Reaktoreingang und der Temperatur am Reaktorausgang), dem Kreislaufvolumenstrom, dem Feed/Recycle Verhältnis, dem Reaktordruck, sofern eine Kühlung vorhanden ist, die Menge an umgewälztem Kühlmittel, der Menge an Produkt im Austrag der ersten Reaktionseinheit, und einer Kombination davon, ausgewählt. Entsprechende Maßnahmen, um diese Parameter einzuregeln, sind dem Fachmann geläufig. In einer besonders bevorzugten Ausführungsform wird der Parameter, der für die Begrenzung des Umsatzes in dem oder in den parallelen Reaktoren der ersten Reaktionseinheit geregelt wird, im Rahmen der vorliegenden Erfindung vorzugsweise aus der Gruppe, bestehend aus der dem mindestens einen Kreislaufreaktor zugeführten Feedmenge (Gesamtmenge aus Frischfeed und Recycle), der Menge an zu hydrierendem Dialkylterephthalat, dem Feed/Recycle Verhältnis und einer Kombination davon, ausgewählt. Dies ist besonders vorteilhaft, weil der Durchsatz der Reaktion deutlich erhöht werden kann, wodurch in kürzerer Zeit mehr Produkt mit geringer Menge an Nebenprodukten gebildet werden können.

Hinsichtlich des Einstellens der Menge an Produkt im Austrag der ersten Reaktionseinheit, als eine Möglichkeit zur Einstellung des Reaktionsumsatzes: Die Begrenzung des Reaktionsumsatzes lässt sich rein rechnerisch auch dadurch erzeugen, dass ein Teil des eingesetzten Feeds um die erste Reaktionseinheit herumgeführt wird, d. h. den oder die Reaktoren der Reaktionseinheit nicht durchfährt und nicht hydriert wird. Der um die erste Reaktionseinheit herumgeführte Teil des Feeds wird dann mit dem Reaktionsaustrag der ersten Reaktionseinheit vermischt, wodurch die Menge an Produkt im Austrag der ersten Reaktionseinheit geregelt wird, und zur zweiten Reaktionseinheit mit dem oder den Reaktor(en), die im geraden Durchgang betrieben wird, geführt. Der zur zweiten Reaktionseinheit geführte Strom weist dann eine Zusammensetzung auf, die der Zusammensetzung eines Reaktionsaustrags aus der ersten Reaktionseinheit entspricht, in der ein Reaktionsumsatz im Bereich von 85 bis 93 %, vorzugsweise 88 bzw. 92 % erzielt worden ist. Dies lässt sich sehr einfach über die Menge des um die erste Reaktionseinheit herumgeführten Feed regeln. In dieser Ausführungsform wird also der gewünschte Umsatz in der ersten Reaktionseinheit nur fingiert. Der wahre Reaktionsumsatz in der ersten Reaktionseinheit kann dann (deutlich) höher liegen und wird durch die Zugabe von Feed künstlich gesenkt.

Das erfindungsgemäße Verfahren könnte grundsätzlich diskontinuierlich oder kontinuierlich durchgeführt werden. Erfindungsgemäß bevorzugt ist es ein kontinuierliches Verfahren. Trotz der Begrenzung des Reaktionsumsatzes in der ersten Reaktionseinheit wird bei dem erfindungsgemäßen Verfahren ein Gesamtumsatz bezogen auf die gesamte Hydriereinheit größer oder gleich 99,7 %, vorzugsweise größer oder gleich 99,8 % erreicht. Die Menge an Nebenprodukten in dem Verfahrensprodukt der Kernhydrierung nach der Hydriereinheit ist vorzugsweise geringer als 1,3 Gew.-%, besonders bevorzugt geringer als 1,2 Gew.-%.

Erfindungsgemäß werden bei der Kernhydrierung Dialkylterephthalate mit C3- bis C16-Alkylgruppen eingesetzt. Die Wahl der Länge der Alkylgruppen ist weniger kritisch und sollte den Fachmann vor keine großen Probleme stellen. Es ist jedoch bevorzugt, dass die beiden Alkylgruppen die gleiche Kettenlänge aufweisen. Das eingesetzte Dialkylterephthalat ist vorzugsweise Dialkylterephthalat mit C4- bis C11-Alkylgruppen, weiterhin bevorzugt ein Dialkylterephthalat mit C4- bis C10-Alkylgruppen, weiterhin bevorzugt ein Dialkylterephthalat mit C5- bis C9-Alkylgruppen, besonders bevorzugt ein Dialkylterephthalat mit C8- oder C9-Alkylgruppen. Ganz besonders bevorzugt ist das eingesetzte Dialkylterephthalat Diethylhexylterephthalat oder Diisononylterephthalat.

Das bei der Kernhydrierung eingesetzte Dialkylterephthalat kann durch Umesterung von Estern der Terephthalsäure beispielsweise Dimethylterephthalat mit einem entsprechenden Alkohol oder durch Veresterung von Terephthalsäure mit einem entsprechenden Alkohol hergestellt werden. Die Kettenlänge des eingesetzten Alkohols entspricht der Kettenlänge im gebildeten Ester.

Erfindungsgemäß werden bei dem erfindungsgemäßen Verfahren zur Kernhydrierung von Dialkylterephthalaten oder allgemein aromatischen Carbonsäureestern heterogene Hydrierkatalysatoren eingesetzt. Das können Katalysatoren, die ein Trägermaterial enthalten, oder ungeträgerte Katalysatoren (ohne Trägermaterial), beispielsweise Raney-Ni sein. Vorzugsweise umfasst der heterogene Hydrierkatalysator, der bei der Kernhydrierung in den mindestens zwei Reaktoren der Hydriereinheit eingesetzt wird, mindestens ein Übergangsmetall auf einem Trägermaterial oder besteht aus mindestens einem Übergangsmetall auf einem Trägermaterial. Geeignete Katalysatoren sind dem Fachmann aber auch geläufig und beispielsweise der WO 03/103830 A1 zu entnehmen.

Das Übergangsmetall des heterogene Hydrierkatalysators ist bevorzugt ein Metall, welches aus der Gruppe 8 des Periodensystems der Elemente (Eisengruppe), vorzugsweise aus der Gruppe, bestehend aus Eisen, Ruthenium, Kobalt, Nickel, Rhodium, Platin, Palladium oder Mischungen davon, ausgewählt wird. Ruthenium ist das in der vorliegenden Erfindung besonders bevorzugte Übergangsmetall des eingesetzten Katalysators. Der Gehalt an Übergangsmetall im heterogenen Hydrierkatalysator liegt vorzugsweise im Bereich von 0,1 bis 10 Gew.-%, vorzugsweise insbesondere im Bereich von 0,2 bis 5 Gew.-%, besonders im Bereich von 0,5 und 3 Gew.-%. Sofern Ruthenium als Übergangsmetall eingesetzt wird, liegt der Rutheniumgehalt, berechnet als Metall, vorzugsweise im Bereich von 0,1 bis 10 Gew.-%, insbesondere im Bereich von 0,2 bis 5 Gew.-%, ganz besonders im Bereich zwischen 0,5 und 3 Gew.-%.

Das Trägermaterial, auf dem sich das Übergangsmetall des heterogenen Hydrierkatalysators befindet, wird vorzugsweise aus der Gruppe, bestehend aus Aktivkohle, Siliciumcarbid, Aluminiumoxid, Siliciumdioxid, Alumosilikat, Zeolithen, Titandioxid, Zirkoniumdioxid, Magnesiumoxid, Zinkoxid oder Mischungen davon, ausgewählt. Bevorzugte Trägermaterialien sind Aluminiumoxid, Siliciumdioxid, Titandioxid und Mischung davon, besonders bevorzugt sind Titandioxid und Aluminiumoxid. Zusätzlich können diese Trägermaterialien Alkalimetall-, Erdalkalimetall- und/oder Schwefel-Komponenten enthalten.

Die heterogenen Hydrierkatalysatoren in den Reaktoren der beiden Reaktionseinheiten können die gleiche oder eine unterschiedliche Zusammensetzung aus Übergangsmetall und Trägermaterial aufweisen. Bevorzugt weisen die mindestens zwei heterogenen Hydrierkatalysatoren die gleiche Zusammensetzung auf, d. h. sie umfassen das gleiche Übergangsmetall und das gleiche Trägermaterial auf, wobei der Gehalt an Übergangsmetall, vorzugsweise der Rutheniumgehalt, unterschiedlich sein kann.

Die Kernhydrierung wird erfindungsgemäß bevorzugt in flüssiger Phase durchgeführt. Eine Kernhydrierung kann mit homogenen, oder heterogenen in suspendierter oder stückig im Festbett angeordneten Hydrierkatalysatoren durchgeführt werden. Im erfindungsgemäßen Verfahren wird eine kontinuierliche Kernhydrierung an einem im Festbett angeordneten heterogenen Katalysator, bei dem sich unter Reaktionsbedingungen die Reaktionsmischung hauptsächlich im flüssigen Zustand befindet, bevorzugt. Vorzugsweise wird/werden der/die Reaktor(en) mit einer Flüssig/Gas-Mischphase, z.B. als Rieselbettreaktor(en) betrieben, der/die vollständig oder teilweise geflutet sein kann/können.

Für die Kernhydrierung können unterschiedliche Verfahrensvarianten gewählt werden. Es kann adiabatisch oder polytrop, ein- oder mehrstufig durchgeführt werden. Im letzteren Fall kann man alle Reaktoren, zweckmäßig Rohrreaktoren, adiabatisch oder polytrop sowie einen oder mehrere adiabatisch und die anderen polytrop betreiben.

Die erfindungsgemäße Kernhydrierung kann in der Flüssig/Gas-Mischphase oder in der Flüssigphase in Dreiphasenreaktoren im Gleichstrom durchgeführt werden, wobei das Hydriergas in an sich bekannter Weise in den flüssigen Edukt/Produktstrom eingebracht wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

In einer bevorzugten Ausführungsform kann das Verfahren zur Kernhydrierung in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen.

Beispielsweise können folgende Stoffe als Lösemittel eingesetzt werden: Geradkettige oder cyclische Ether, wie beispielsweise Tetrahydrofuran oder Dioxan sowie aliphatische Alkohole, in denen der Alkylrest 1 bis 13 Kohlenstoffatome aufweist. Bevorzugt verwendbare Alkohole oder Alkoholgemische sind beispielsweise Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch ist die Nebenproduktbildung durch Umesterung ausgeschlossen. Ein weiteres bevorzugtes Lösemittel ist das Hydrierprodukt, hier also die kernhydrierten Dialkylterephthalate selbst.

Die Kernhydrierung kann in einem Druckbereich von 3 bis 300 bar, vorzugsweise 20 bis 200 bar durchgeführt werden. Die Hydriertemperaturen liegen vorzugsweise im Bereich von 50 bis 250°C, insbesondere im Bereich von 60 bis 200 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Neben Wasserstoff können beispielsweise COz, Stickstoff oder Methan im Hydriergas vorhanden sein. Bevorzugt wird Wasserstoff in einer Reinheit von größer 95 %, insbesondere größer 98 % eingesetzt.

Die Erfindung wird nachfolgend anhand von Beispielen erläutert. Diese Beispiele zeigen exemplarische Ausführungsformen und sind nicht einschränkend zu verstehen.

### Beispiel

Es wurden Hydrierversuche mit Diisononylphthalat (DINP) und Diisononylterephthalat (DINT) in einer Hydriereinheit, die aus einem Kreislaufreaktor und einem im geraden Durchgang betriebenen Reaktor bestand, wie folgt durchgeführt:
Die Kernhydrierung von DINT bzw. DINP erfolgte in einem Rohrreaktor im Kreislaufbetrieb mit einem angeschlossenen zweiten Rohrreaktor im geraden Durchgang. Die Rohrreaktoren wurden im Gleichstrom mit Flüssigphase (DINT bzw. DINP und Hydrierprodukt) und Gasphase (Wasserstoff) im Rieselbett durchströmt. Als Hydrierkatalysator wurde ein handelsüblicher Rutheniumkatalysator (Specialyst^{®} 102: 1% Ru auf einem TiO₂-Träger, Fa. Evonik Operations GmbH) in beiden Reaktoren verwendet. Dieser wurde in dem Kreislaufrohrreaktor mit einem Innendurchmesser von 40 mm und einer Länge von 479 mm und im zweiten Reaktor mit einem Innendurchmesser von 20 mm und einer Länge von 1076 mm eingesetzt. Die in der Kernhydrierung eingesetzte Feedrate an DINT bzw. DINP wurde je nach Versuch zwischen ca. 180 - 800 g/h variiert, der Kreislaufstrom betrug stets 80 L/h. Die Feedrate wurde während der Reaktion geändert, um dadurch Einfluss auf den Reaktionsumsatz zu nehmen. Die Wasserstoffregelung erfolgte über eine konstante Abgasfahrweise mit einem Abgasstrom von 0,5 L/h. Die Versuche wurden jeweils bei einem Anlagendruck von 100 bar sowie einer Rohrreaktortemperatur von 105 °C im Kreislaufreaktor und 110 °C im zweiten Reaktor durchgeführt. Der Austrag aus der Hydriereinheit wurde auf die Anwesenheit und die Menge an Nebenprodukten mittels Gaschromatographie (GC) untersucht. Die Ergebnisse sind in der nachfolgenden Tabelle 1 gezeigt:

| **Edukt** | **Temperatur / °C** | **Feederate / g/h** | **Umsatz im Kreislaufreaktor / %** | **Gesamtumsatz (nach letzen Reaktor) / %** | **Menge an Nebenprodukten nach letzem Reaktor / Gew.- %** |
|---|---|---|---|---|---|
| DINT | 105 | 246 | 95,1 | > 99,8 | 1,49 |
| DINT | 105 | 397 | 91,0 | > 99,8 | 1,11 |
| DINT | 105 | 429 | 90,0 | > 99,8 | 1,02 |
| DINP | 105 | 427 | 94,9 | > 99,8 | 0,49 |
| DINP | 105 | 792 | 90,1 | > 99,8 | 0,43 |

Es zeigt sich, dass bei der Kernhydrierung des Terephthalats durch Begrenzung des Umsatzes im ersten Reaktor die Menge an Nebenprodukten nach dem letzten Reaktor signifikant verringert werden kann. Bei dem entsprechenden Phthalat ist diese Änderung hingegen nur minimal. Die Begrenzung des Umsatzes beim Terephthalat hat hier weiterhin den Vorteil, dass die Feedrate dafür deutlich erhöht werden kann. Es kann also mehr kernhydriertes Produkt bei höherer Reinheit hergestellt und somit die Wirtschaftlichkeit des Verfahrens erhöht werden

## Patentansprüche

1. Verfahren zur Kernhydrierung von Dialkylterephthalaten mit C3- bis C16-Alkylgruppen, vorzugsweise Dialkylterephthalaten mit C4- bis C11-Alkylgruppen, zu den 1,4-Cyclohexandicarbonsäureestern mit den entsprechenden Alkylgruppen in einer Hydriereinheit aus zwei hintereinandergeschalteten Reaktionseinheiten, die aus einem Reaktor oder mehreren parallelen Reaktoren bestehen, wovon der oder die Reaktor(en) in der zweiten Reaktionseinheit im geraden Durchgang betrieben werden, wobei in den Reaktoren der beiden Reaktionseinheiten der Hydriereinheit jeweils ein heterogener Hydrierkatalysator vorliegt und wobei das Verfahren sich dadurch auszeichnet, dass
der eingesetzte aromatische Carbonsäureester als Feed in den Reaktoren der Reaktionseinheiten mit einem Wasserstoff-enthaltenden Gas in Kontakt gebracht wird, wobei der Reaktionsumsatz im in der ersten Reaktionseinheit überwacht wird, und
wobei mindestens ein Parameter in Bezug zur ersten Reaktionseinheit, ausgewählt aus der Gruppe, bestehend aus der der ersten Reaktionseinheit zugeführten Feedmenge (Gesamtmenge aus Frischfeed und Recycle), der Menge an zu hydrierendem Dialkylterephthalat, der Reaktortemperatur in dem oder den Reaktoren der ersten Reaktionseinheit, dem Temperaturanstieg, dem Kreislaufvolumenstrom, dem Feed/Recycle Verhältnis, dem Verhältnis von Wasserstoff zu aromatischem Carbonsäureester, dem Reaktordruck, der Feedzusammensetzung, der Katalysatormenge, der Katalysatorzusammensetzung, sofern eine Kühlung vorhanden ist, die Menge an umgewälztem Kühlmittel oder der Menge an Produkt im Austrag der ersten Reaktionseinheit und eine Kombination davon so geregelt wird, dass ein Reaktionsumsatz im Bereich von 85 bis 93 %, vorzugsweise 88 bis. 92 % erzielt wird.

2. Verfahren nach Anspruch 1, wobei in der ersten Reaktionseinheit ein Reaktor vorhanden ist, der im Kreislauf betrieben wird.

3. Verfahren nach Anspruch 1 und 2, wobei die Menge an Nebenprodukten in dem Verfahrensprodukt der Kernhydrierung nach der Hydriereinheit geringer als 1,3 Gew.-%, vorzugsweise geringer als 1,2 Gew.-% ist.

4. Verfahren nach einem der vorhergehenden Ansprüche, wobei der Gesamtumsatz bei dem Verfahren bezogen auf die gesamte Hydriereinheit größer oder gleich 99,7 %, vorzugsweise größer oder gleich 99,8 % ist.

5. Verfahren nach einem der vorhergehenden Ansprüche, wobei das bei der Kernhydrierung eingesetzte Dialkylterephthalat ein Dialkylterephthalaten mit C4- bis C10-Alkylgruppen, vorzugsweise ein Dialkylterephthalat mit C5- bis C9-Alkylgruppen, besonders bevorzugt ein Dialkylterephthalat mit C8- oder C9-Alkylgruppen ist.

6. Verfahren nach Anspruch 5, wobei das bei der Kernhydrierung eingesetzte Dialkylterephthalat durch Umesterung von Dimethylterephthalat mit einem Alkohol mit 4 bis 10 Kohlenstoffatomen oder durch Veresterung von Terephthalsäure mit einem Alkohol mit 4 bis 10 Kohlenstoffatomen hergestellt wird.

7. Verfahren nach Anspruch 5, wobei der bei der Kernhydrierung eingesetzte Dialkylterephthalat Diethylhexylterephthalat oder Diisononylterephthalat ist.

8. Verfahren nach einem der vorhergehenden Ansprüche, wobei der bei der Kernhydrierung in den Reaktoren der zwei Reaktoreinheiten der Hydriereinheit eingesetzte heterogene Hydrierkatalysator ein Übergangsmetall auf einem Trägermaterial umfasst.

9. Verfahren nach Anspruch 8, wobei das Übergangsmetall ein Metall der Gruppe 8 des Periodensystems der Elemente (Eisengruppe), vorzugsweise Ruthenium ist.

10. Verfahren nach Anspruch 8 oder 9, wobei das Trägermaterial aus der Gruppe, bestehend aus Aktivkohle, Siliciumcarbid, Aluminiumoxid, Siliciumdioxid, Alumosilikat, Zeolithen, Titandioxid, Zirkoniumdioxid, Magnesiumoxid, Zinkoxid oder Mischungen davon, ausgewählt wird.

11. Verfahren nach Anspruch 10, wobei das Trägermaterial Titandioxid oder Aluminiumoxid ist.

12. Verfahren nach einem der Ansprüche 8 bis 11 wobei der Gehalt an Übergangsmetall im heterogenen Hydrierkatalysator im Bereich von 0,1 bis 10 Gew.-%, vorzugsweise insbesondere im Bereich von 0,2 bis 5 Gew.-%, besonders im Bereich von 0,5 und 3 Gew.-% liegt.

13. Verfahren nach einem der Ansprüche 8 bis 12, wobei die Hydrierkatalysatoren in den mindestens zwei Reaktoren der Hydriereinheit die gleiche Zusammensetzung aufweisen.

14. Verfahren nach einem der vorhergehenden Ansprüche wobei die Hydriertemperatur bei der Kernhydrierung im Bereich von 50 bis 250 °C liegt.

15. Verfahren nach einem der vorhergehenden Ansprüche, wobei die Kernhydrierung in einem Druckbereich von 3 bis 300 bar durchgeführt wird.

## Claims

1. Process for ring hydrogenation of dialkyl terephthalates having C3- to C16-alkyl groups, preferably dialkyl terephthalates having C4- to C11-alkyl groups, to give 1,4-cyclohexanedicarboxylic esters having the corresponding alkyl groups, in a hydrogenation unit consisting of two reaction units connected in series, which consist of one reactor or two or more parallel reactors, of which the reactor(s) in the second reaction unit are operated in a straight pass, wherein a heterogeneous hydrogenation catalyst is present in the reactors of each of the two reaction units of the hydrogenation unit and wherein the process is **characterized in that**
the aromatic carboxylic ester used as feed to the reactors of the reaction units is brought into contact with a hydrogen-containing gas, wherein the reaction conversion in the first reaction unit is monitored, and
wherein at least one parameter in relation to the first reaction unit, selected from the group consisting of the amount of feed supplied to the first reaction unit (total amount of fresh feed and recycle), the amount of dialkyl terephthalate to be hydrogenated, the reactor temperature in the reactor(s) of the first reaction unit, the temperature increase, the circulation volume flow, the feed/recycle ratio, the ratio of hydrogen to aromatic carboxylic ester, the reactor pressure, the feed composition, the amount of catalyst, the catalyst composition, if cooling is present, the amount of coolant circulated or the amount of product in the discharge of the first reaction unit and a combination thereof, is controlled such that a reaction conversion in the range of 85 to 93%, preferably 88 to 92%, is achieved.

2. Process according to Claim 1, wherein a reactor is present in the first reaction unit, which is operated in a closed loop.

3. Process according to Claims 1 and 2, wherein the amount of by-products in the ring hydrogenation process product after the hydrogenation unit is less than 1.3% by weight, preferably less than 1.2% by weight.

4. Process according to any of the preceding claims, wherein the total conversion in the process, based on the total hydrogenation unit, is greater than or equal to 99.7%, preferably greater than or equal to 99.8%.

5. Process according to any of the preceding claims, wherein the dialkyl terephthalate used in the ring hydrogenation is a dialkyl terephthalate having C4- to C10-alkyl groups, preferably a dialkyl terephthalate having C5- to C9-alkyl groups, particularly preferably a dialkyl terephthalate having C8- or C9-alkyl groups.

6. Process according to Claim 5, wherein the dialkyl terephthalate used in the ring hydrogenation is prepared by transesterification of dimethyl terephthalate with an alcohol having 4 to 10 carbon atoms or by esterification of terephthalic acid with an alcohol having 4 to 10 carbon atoms.

7. Process according to Claim 5, wherein the dialkyl terephthalate used in the ring hydrogenation is diethylhexyl terephthalate or diisononyl terephthalate.

8. Process according to any of the preceding claims, wherein the heterogeneous hydrogenation catalyst used in the ring hydrogenation in the reactors of the two reactor units of the hydrogenation unit comprises a transition metal on a support material.

9. Process according to Claim 8, wherein the transition metal is a metal from Group 8 of the Periodic Table of the Elements (iron group), preferably ruthenium.

10. Process according to Claim 8 or 9, wherein the support material is selected from the group consisting of activated carbon, silicon carbide, aluminium oxide, silicon dioxide, aluminosilicate, zeolites, titanium dioxide, zirconium dioxide, magnesium oxide, zinc oxide or mixtures thereof.

11. Process according to Claim 10, wherein the support material is titanium dioxide or aluminium oxide.

12. Process according to any of Claims 8 to 11, wherein the transition metal content in the heterogeneous hydrogenation catalyst is in the range from 0.1% to 10% by weight, preferably particularly in the range from 0.2% to 5% by weight, particularly in the range from 0.5% to 3% by weight.

13. Process according to any of Claims 8 to 12, wherein the hydrogenation catalysts in the at least two reactors of the hydrogenation unit have the same composition.

14. Process according to any of the preceding claims, wherein the hydrogenation temperature in the ring hydrogenation is in the range from 50 to 250°C.

15. Process according to any of the preceding claims, wherein the ring hydrogenation is carried out in a pressure range of 3 to 300 bar.

## Revendications

1. Procédé pour l'hydrogénation au noyau de téréphtalates de dialkyle comportant des groupes alkyle en C₃ à C₁₆, de préférence téréphtalates de dialkyle comportant des groupes alkyle en C₄ à C₁₁, en les esters d'acides 1,4-cyclohexanedicarboxyliques comportant les groupes alkyle correspondants, dans une unité d'hydrogénation constituée de deux unités de réaction montées en série qui consistent en un réacteur ou plusieurs réacteurs parallèles, dont le ou les réacteur(s) dans la seconde unité de réaction est/sont utilisé(s) en passage direct, un catalyseur d'hydrogénation hétérogène étant chaque fois présent dans les réacteurs des deux unités de réaction de l'unité d'hydrogénation et le procédé **se caractérisant par le fait que**
l'ester d'acide carboxylique aromatique utilisé est mis en contact en tant que charge dans les réacteurs des unités de réaction avec un gaz contenant de l'hydrogène, le degré d'avancement de la réaction étant surveillé dans la première unité de réaction, et
au moins un paramètre en rapport avec la première unité de réaction, choisi dans le groupe constitué par la quantité de charge amenée à la première unité de réaction (quantité totale de charge neuve et recyclage), la quantité de téréphtalate de dialkyle à hydrogéner, la température de réacteur dans le ou les réacteurs de la première unité de réaction, l'élévation de température, le débit volumique de circuit, le rapport charge/recyclage, le rapport d'hydrogène à ester d'acide carboxylique aromatique, la pression dans le réacteur, la composition de la charge, la quantité de catalyseur, la composition du catalyseur, dans la mesure où un refroidissement est présent, la quantité de réfrigérant mis en circulation ou la quantité de produit dans la décharge de la première unité de réaction et une combinaison de tels paramètres, est réglé de telle façon qu'est atteint un rendement de réaction dans la plage de 85 à 93 %, de préférence 88 à 92 %.

2. Procédé selon la revendication 1, dans lequel dans la première unité de réaction est présent un réacteur qui est utilisé en circuit.

3. Procédé selon la revendication 1 et la revendication 2, dans lequel la quantité de sousproduits dans le produit du procédé de l'hydrogénation au noyau après l'unité d'hydrogénation est inférieure à 1,3 % en poids, de préférence inférieure à 1,2 % en poids.

4. Procédé selon l'une des revendications précédentes, dans lequel le rendement global dans le procédé sur la base de l'unité d'hydrogénation dans son ensemble est supérieur ou égal à 99,7 %, de préférence supérieur ou égal à 99,8 %.

5. Procédé selon l'une des revendications précédentes, dans lequel le téréphtalate de dialkyle utilisé dans l'hydrogénation au noyau est un téréphtalate de dialkyle comportant des groupes alkyle en C₄ à C₁₀, de préférence un téréphtalate de dialkyle comportant des groupes alkyle en C₅ à C₉, de façon particulièrement préférée un téréphtalate de dialkyle comportant des groupes alkyle en C₈ ou C₉.

6. Procédé selon la revendication 5, dans lequel le téréphtalate de dialkyle utilisé dans l'hydrogénation au noyau est préparé par transestérification de téréphtalate de diméthyle avec un alcool ayant de 4 à 10 atomes de carbone ou par estérification d'acide téréphtalique avec un alcool ayant de 4 à 10 atomes de carbone.

7. Procédé selon la revendication 5, dans lequel le téréphtalate de dialkyle utilisé dans l'hydrogénation au noyau est le téréphtalate de diéthylhexyle ou le téréphtalate de diisononyle.

8. Procédé selon l'une des revendications précédentes, dans lequel le catalyseur d'hydrogénation hétérogène utilisé dans l'hydrogénation au noyau dans les réacteurs des deux unités de réaction de l'unité d'hydrogénation comprend un métal de transition sur une matière de support.

9. Procédé selon la revendication 8, dans lequel le métal de transition est un métal du groupe 8 du système périodique des éléments (groupe du fer), de préférence le ruthénium.

10. Procédé selon la revendication 8 ou 9, dans lequel la matière de support est choisie dans le groupe constitué par le charbon actif, le carbure de silicium, l'oxyde d'aluminium, le dioxyde de silicium, un aluminosilicate, les zéolithes, le dioxyde de titane, le dioxyde de zirconium, l'oxyde de magnésium, l'oxyde de zinc ou des mélanges de ceux-ci.

11. Procédé selon la revendication 10, dans lequel la matière de support est le dioxyde de titane ou l'oxyde d'aluminium.

12. Procédé selon l'une des revendications 8 à 11, dans lequel la teneur en métal de transition du catalyseur d'hydrogénation hétérogène se situe dans la plage de 0,1 à 10 % en poids, de préférence en particulier dans la plage de 0,2 à 5 % en poids, en particulier dans la plage de 0,5 à 3 % en poids.

13. Procédé selon l'une des revendications 8 à 12, dans lequel les catalyseurs d'hydrogénation présentent la même composition dans lesdits au moins deux réacteurs de l'unité d'hydrogénation.

14. Procédé selon l'une des revendications précédentes, dans lequel la température d'hydrogénation dans l'hydrogénation au noyau se situe dans la plage de 50 à 250 °C.

15. Procédé selon l'une des revendications précédentes, dans lequel l'hydrogénation au moyeu est effectuée dans une plage de pression de 3 à 300 bars.
